(19) Europäisches Patentamt

European Patent Office

Office européen des brevets

(11) Publication number: **0 313 184 B1**

(12) **EUROPEAN PATENT SPECIFICATION**

(45) Date of publication of patent specification: **17.06.92**

(51) Int. Cl.⁵: **B26B 21/44**, A61K 7/15

(21) Application number: **88305434.8**

(22) Date of filing: **15.06.88**

(54) **Shaving aid and razor component provided with a shaving aid.**

(30) Priority: **21.10.87 US 111956**

(43) Date of publication of application:
**26.04.89 Bulletin 89/17**

(45) Publication of the grant of the patent:
**17.06.92 Bulletin 92/25**

(84) Designated Contracting States:
**AT BE CH DE ES FR GB GR IT LI LU NL SE**

(56) References cited:
**US-A- 2 145 583**
**US-A- 4 170 821**

**Wörterbuch der Kosmetik**
**(Wissenschaftliche Verlagsgesellschaft mbH**
**Stuttgart, veröffentlich 1985)**

(73) Proprietor: **WARNER-LAMBERT COMPANY**
**201 Tabor Road**
**Morris Plains New Jersey 07950(US)**

(72) Inventor: **Vreeland, William Elbert**
**148 Dickenson Drive**
**Shelton Connecticut 06484(US)**
Inventor: **Williams, Peter Stephen**
**985 James Farm Road**
**Stratford Connecticut 06497(US)**

(74) Representative: **Coxon, Philip et al**
**Eric Potter & Clarkson St. Mary's Court St.**
**Mary's Gate**
**Nottingham NG1 1LE(GB)**

## Description

This invention relates to a shaving aid, and to a razor component provided with a shaving aid.

In recent years the concept of delivering a shave-aiding agent in the form of a shaving lubricant from a microporous plastic strip attached to a razor head has gained increasing popularity. The lubricant, which is usually polyethylene oxide, is water soluble and is deposited on the face during the act of shaving.

It is known, for example, that shavers tend to shave repeatedly in the same area. The initial razor pass removes the foam or shaving cream and a substantial percentage of the whiskers. Repeated passes remove the remainder of the whiskers and are the primary source of irritation; this irritation is substantially eliminated by the presence of a lubricant delivered to the face during the act of shaving, rather than before the shaving. Shaving foam or lather introduce lubricants before shaving commences, while polyethylene oxide delivered from a microporous substrate liberates lubricating material during each stroke of the razor. This second lubrication minimizes the facial irritation which occurs during "second-stroke shaving." (The term second-stroke shaving refers to shaving strokes which occur in an area of the face after the lather initially covering that area has been removed by shaving.)

Such a lubricating and delivery system is disclosed in US-A-4,170,821. As disclosed in this patent, the polyethylene oxide can be added directly to the face through the leaching of the pure compound or by leaching of the compound which had been admixed with a thermoplastic substrate. The shaving aid is in the form of an essentially rigid thermoplastic super-structure including microporous apertures throughout the surface containing polyethylene oxide. These admixtures can be formed, as disclosed in US-A-4,170,821, in a variety of ways such as extrusion or injection moulding the admixture.

This patent also suggests the use of a blood coagulant for suppression of bleeding that occurs from nicks and cuts as another additive useful alone or in combination with a cosmetic agent, a medicinal agent, a cleaning agent, an agent which modifies the chemical structure of the hair or lubricating agents of various types, both water soluble and water insoluble.

It has now been found that the combination of polyethylene oxide and a water soluble astringent for simultaneous dispensing during the act of shaving, minimizes the perception of nicks associated with shaving by delivering astringents essentially simultaneously with the nicking resulting from shaving, while essentially eliminating the burning or stinging sensation associated with their addition.

Thus, according to one aspect of the invention there is provided a shaving aid according to the features of Claim 1.

The astringent is preferably aluminium sulfate.

Desirably the astringent is present at a level not greater than about 20% by weight of the shaving aid.

In a preferred embodiment the shaving aid comprises a microporous retaining structure which acts as a delivery vehicle for the polyethylene oxide/astringent combination.

The retaining structure is preferably a water insoluble thermoplastics material.

According to another aspect of the invention there is provided a razor blade cover, characterised in that said cover is provided with a shaving aid as described above.

According to another aspect of the invention there is provided a razor head characterised by a blade cover, at least one blade, the or each blade having a blade cutting edge, and a blade support having a guard bar disposed forwardly of the blade cutting edge, wherein the blade cover and/or the guard bar is provided with a shaving aid as described above.

The invention also provides a method of making a shaving aid according to the invention comprising forming a mixture of a thermoplastics material, polyethylene oxide and an astringent, and subjecting said mixture to a forming step selected from extrusion, injection moulding and compression moulding to produce a delivery vehicle which comprises a microporous structure of thermoplastics material containing the polyethylene oxide and the astringent.

The concept of this invention utilizes the mixture of a lubricant useful for reducing razor drag, such as polyethylene oxide, in combination with a water soluble astringent. This combination is delivered from a razor head during the act of shaving making the astringent instantly available while minimizing the visible evidence of nicks and the stinging and burning sensation associated with the application of an astringent after the nick has been perceived by the shaver.

As used throughout this specification, the razor head is defined to include both a cartridge which is disposable and mounted on a permanent handle, and the portion of a disposable razor which corresponds to the cartridge. It is immaterial for purposes of the invention whether the polyethylene oxide lubricant and the astringent are delivered from the head of a disposable razor or from a cartridge attached to a permanent handle.

The razor head may have either a single blade, or two blades with the shaving edges in staggered relationship to each other. When two blades are provided, the blades may be separated by a spacer or by other means, such as utilizing blades having holes of different construction which correlate to indentations on posts depending upward from the seat, downward from the cap, or both.

The razor head usually includes a guard bar which directs the flow of skin to the blade or blades, a blade support from which the guard bar depends and which supports one of the blades, and a blade cover for protecting the blade or blades and/or for maintaining the blades in a predetermined vertical and/or horizontal configuration.

The razor head also may include a back formed from either the cover or the support and a set of cams formed as part of the bottom of the blade support. It is immaterials for purposes of this invention whether the razor head has one or two blades, is designed to be pivoting by use of the above-referred to cams, or how the blades are mounted within the razor head.

Reference is now made to the accompanying drawing which is a perspective view of a razor head according to the invention.

In the drawing a razor head 10 includes a blade 18, a blade cover in the form of a cap 22 and a blade support in the form of a seat 12. A guard bar 14 extends from the seat 12 and the bottom portion of the seat 12 includes a channel 20 which is designed to engage a suitable mating handle for use during shaving. A shaving aid in the form of a lubricating strip 24 is positioned in a recess 26 mounted on cap 22. The lubricating strip 24 can be adhesively bound to the cap 22 or can be affixed in place by the use of fold over tabs (not shown) or the like. It is also possible, or course, to utilize a shaving aid configuration other than the strip 24, but it is preferred that the shaving aid is positioned on the cap 22.

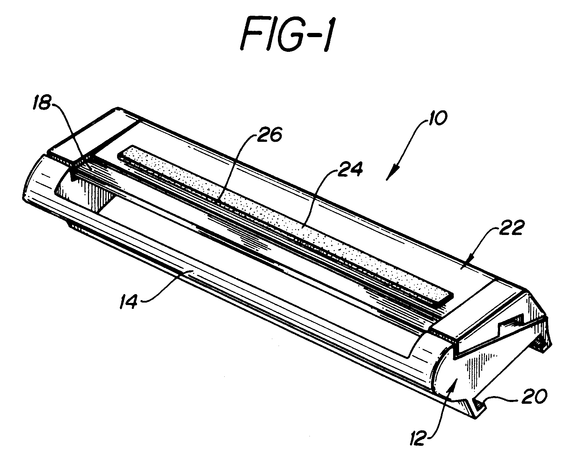

The lubricating strip 24 is defined as a strip which includes polyethylene oxide. The strip shown in figure 1 is formed of a retaining structure in the form of a water insoluble thermoplastic material, which a polyethylene oxide shave-aiding agent present as an admixture during the forming process (as described in US-A-4,170,821 directed towards the forming of microporous vehicles).

The formation of the thermoplastic delivery vehicle as a means for discharging the leachable substrate is preferred for several reasons, among them that the shaving geometry will not be altered because the retaining structure of the strip is formed from a water insoluble material rather than a water soluble material.

The presence of an astringent, preferably substantially uniformly dispersed throughout the polyethylene oxide matrix, produces a combination which, unexpectedly, substantially decreases the visible evidence of nicks while eliminating the discomfort associated with the provision of an astringent.

Astringents useful in this invention must, of course, be water soluble and readily dispersible throughout a polyethylene oxide carrier. Some astringents such as those based upon trivalent chromium are not preferred because of the possibility of skin reaction associated with chromium. While this possibility is somewhat remote due to the extremely low concentrations ultimately employed in the skin, more satisfactory astringents such as those based on trivalent aluminium salts are readily available. Amongst the trivalent salts, aluminium sulfate, which is a major component of styptic pencils, is particularly preferred.

Astringents such as aluminium sulfate are added at levels sufficient to substantially eliminate the visible evidence of minor nicks during shaving, generally in the area of 5% by weight of the polyethylene oxide containing thermoplastic strip. The maximum amount of aluminium sulfate is determined by process limitations when a microporous substrate is used as the delivery system. Amounts of astringent greater than 15% by weight of the polyethylene oxide containing thermoplastic strip are generally not desirable because of the difficulties inherent in blending during the extrusion step sometimes utilized for the manufacture of the microporous combination.

While total amounts of aluminium sulfate or other astringents may vary depending on the process it is generally preferred to keep the level below about 20%.

It is believed that the uniform overall dispersion resulting from the delivery of the astringent in the polyethylene oxide provides for the essentially instant stopping of blood flow before blood resulting from razor nicks is visually apparent. It is also believed that the uniform, even dispersion of polyethylene oxide eliminates the stinging associated with localized essentially pure concentrations of astringent associated with styptic pencils. In any event, the combination has proven surprisingly useful in maximizing the advantages associated with polyethylene oxide and astringents.

Equally surprising, is the lack of effect on the stability of the polyethylene oxide lubricant due to the addition of an astringent such as aluminium sulfate. A mixture of polethylene oxide of two molecular weights and aluminium sulfate present at a level of 10% by weight of the polyethylene oxide strip combination was blended, formed and stored for 71 days with a periodic viscosity measurements indicating virtually no change in viscosity of the combination containing aluminium sulfate when compared with an identical combination without the aluminium sulfate being present. Viscosity in these systems is used as an indicator of stability as required for shelf-stable, storage and use.

Example 1

A subjective shaving test was designed involving 100 males over the age of 18. Subjects chosen for this test were those who claimed to nick sometimes or always when they shave. Each participant completed 4 shaves with each of the participants shaving with a standard Super II Plus cartridge (Super II is a trade mark of the Schick group of the Warner-Lambert Company, Morris Plains, New Jersey) and a Super II Plus razor in which the polyethylene oxide containing strip included 10% aluminium sulfate by weight of the polyethylene oxide containing strip. Each of the shavers shaved for 4 days with one of the razor samples and then for 4 days with the other razor sample. The order of first use was divided equally between the 100 subjects. Of the 86 subjects responding, 53% preferred the standard polyethylene oxide formulation while 47% preferred the formulation with aluminium sulfate. By statistical analysis these two percentages were found to be at parity at the 95% confidence level. There were no significant differences found by those preferring either sample with regard to complaints or evidence of satisfaction or dissatisfaction.

It should be noted that this test was designed strictly as a shaving preference test with no attempt made to educate the users as to the effect of aluminium sulfate. No attempt was made to measure the amount of nicks occuring with the shave.

The general formulation used for this example included:

| Ingredient | % by weight |
|---|---|
| Medium impact polystyrene | 28.9 |
| Polyethylene oxide | 61 |
| BHT | 0.1 |
| $Al_2(SO_4)_3$ | 10 |

The control differed from the above example in that the amount of medium impact polystyrene was 38.9%.

The Super II Plus razor referred to above, utilizes an extruded strip in both the control and comparison containing aluminium sulfate; the extruded strip was used to form the microporous vehicle utilizing polystyrene as the structural retaining component. While the microporous strip can be formed by extrusion, injection moulding, or compression, the percentages of the various components differ because of the relative ease or difficulty in liberation of the polyethylene oxide when the various processes are used. Lower levels of polyethylene oxide are employed where, as is the case with extrusion, they can be more completely liberated. For this reason, extrusion is the currently preferred method of manufacture. Extrusion is also a simpler process when compared to injection moulding.

The preferred ranges of the components associated with the different processes referred to above are set out in the table below.

Table I

| Process | Aluminium Sulfate $Al_2(SO_4)_3$ | Polyethylene Oxide |
|---|---|---|
| Extrusion | 5% | 60-75% |
| Extrusion | 15% | 50-65% |
| Compression moulding | 3% | 47-62% |
| Compression moulding | 15% | 40-55% |
| Injection moulding | 5% | 65-80% |
| Injection moulding | 15% | 55-70% |

As can be seen from the above values, the amount of aluminium sulfate or other astringent dictates the level of polyethylene oxide chosen. In all instances the percentages are by weight, with the remainder of the formulation made up of polystyrene preferably of the medium impact type.

Particularly preferred levels for each process are indicated in Table II below.

4

Table II

| Process | Polystyrene | Aluminium Sulfate $Al_2(SO_4)_3$ | Polyethylene Oxide | BHT |
|---|---|---|---|---|
| Extrusion | 28.9 | 10 | 61 | 0.1 |
| Compression moulding | 40 | 5 | 55 | |
| Injection moulding | 19.9 | 10 | 70 | 0.1 |

In all instances, it is preferred that the polyethylene oxide has a molecular weight greater than 100,000.

Example 2

Two shaving aid formulations were prepared containing medium impact polystyrene, aluminium sulfate and polyethylene oxide. One of the formulations was identical to that set forth in Example 1, while the second formulation reduced the amount of polystyrene by 5% to a level of 23.9% and increased the level of polyethylene oxide from 61 to 66%. These samples were compared to a sample containing no aluminium sulfate in a subjective test conducted over 4 days. In this test a person is asked to shave one side of his face with one sample and the other side with a second sample. One the second day the process is reversed. On the third day the products were changed and the second formulation having increased polyethylene oxide was utilized in the same manner as day one and then the sides of the face were shaved in reverse order on day four.

Comparisons were gathered between samples having differing levels of polyethylene oxide while aluminium sulfate was maintained at the same level and these samples were compared to the formulation containing polyethylene oxide only. Forty four males were utilized for this test and only the subjects who showed nicking were evaluated. The total number of nicks were determined by immediately patting the side of the face shaved with a tissue and counting the number of blood spots. The data was then evaluated with the results being a directional indicator of superior resistance to nicking due to the presence of aluminium sulfate. The indication is directional because of the size of the sample rather than any failure in number. Table III below summarizes the blood spot data.

A nick, for purposes of this invention is defined as any skin damage which will produce a blood spot under the test conditions set forth above.

Table III

| Number of Blood Spots | | | |
|---|---|---|---|
| $Al_2(SO_4)_3$ (61%PEO) | Schick Super II Plus without aluminium sulfate | Aluminium Sulfate (66% PEO) | Schick Super II Plus without aluminium sulfate |
| 4.2 | 7.1 | 8.5 | 10.3 |

Example 3

This example involves the preparation of a microporous strip by compression. A formulation having the components and at the weights indicated below was mixed using the P-K Twin Shell Blender made by Patterson-Kelly, East Stroudsburg, PA., for 30 minutes.

| Formulation | Weight/percent |
|---|---|
| Polyethylene oxide | 55% |
| Polystyrene | 40% |
| $Al_2(SO_4)_3$ | 5% |
| Total | 100% |

After blending, the material was compressed without heat into inserts of the same approximate shape of the standard extruded insert in an Acromark Press Model 630-50, Berkeley Heights, NJ for 1 to 2 seconds at a pressure at a machine setting of 50 psi (345kPa). This product was compared to a standard Super II cartridge without the polyethylene oxide lubricant being present. Out of 38 subjects who nicked, the average number of nicks for the polyethylene oxide-aluminium sulfate-containing razor was 6.0 while the standard Super II showed a value of 8.9 nicks per full face shave. This data is statistically significant at the 95% confidence level.

It is apparent from the examples set out above that the presence of an astringent such as aluminium sulfate does not interfere with the lubricity desired from polyethylene oxide. It is also clear that a shave having significant fewer nicks associated with it results from the combination of polyethylene oxide and aluminium sulfate and, apparently, the combination is comparable to the polyethylene oxide razor without astringent for overall comfort.

## Claims

1. A shaving aid (24) which is solid and adapted to be attached to a razor head (10) and including a shaving lubricant of polyethylene oxide, characterised in that a trivalent aluminium salt is added as a water soluble astringent such that the shaving aid substantially decreases the visible evidence of nicks while eliminating the discomfort associated with the provision of an astringent alone.

2. A shaving aid according to claim 1 characterised in that the astringent is aluminium sulfate.

3. A shaving aid according to claim 1 or 2 characterised in that the astringent is present at a level not greater than about 20% by weight of the shaving aid.

4. A shaving aid according to any preceding claim, further characterised by a microporous retaining structure which acts as a delivery vehicle for the polyethylene oxide/astringent combination.

5. A shaving aid according to claim 4, characterised in that the retaining structure is a water insoluble thermoplastics material.

6. A shaving aid according to any preceding claim, which is in the form of a strip.

7. A razor blade cover, characterised in that said cover is provided with a shaving aid according to any preceding claim.

8. A razor blade cover according to claim 7, characterised in that the shaving aid is provided on an upper surface of the cover.

9. A razor head characterised by a blade cover, at least one blade, the or each blade having a blade cutting edge, and a blade support having a guard bar disposed forwardly of the blade cutting edge, wherein the blade cover and/or the guard bar is provided with a shaving aid according to any of claims 1 to 6.

10. A method of making a shaving aid according of any of claims 1 to 6 comprising forming a mixture of a thermoplastics material, polyethylene oxide and an astringent, and subjecting said mixture to a forming step selected from extrusion, injection moulding and compression moulding to produce a delivery vehicle which comprises a microporous structure of thermoplastics material containing the polyethylene oxide and the astringent.

## Revendications

1. Un produit d'aide au rasage (24) qui est solide et susceptible d'être fixé à une tête de rasage (10) et comporte un lubrifiant de rasage à l'oxyde de polyéthylène, caractérisé en ce qu un sel d'aluminium trivalent est ajouté comme astringent soluble dans l'eau de telle façon que le produit d'aide au rasage diminue de façon sensible la visibilité des coupures tout en éliminant l'inconfort associé au fait de prévoir un astringent isolé.

2. Un produit d'aide au rasage selon la revendication 1, caractérisé en ce que l'astringent est du sulfate d'aluminium.

3. Un produit d'aide au rasage selon la revendication 1 ou 2, caractérisé en ce que l'astringent est présent à un niveau qui n'est pas supérieur à 20% en poids du produit d'aide au rasage.

4. Un produit d'aide au rasage selon l'une quelconque des revendications précédentes, caractérisé en outre par une structure microporeuse de retenue qui agit comme véhicule de distribution pour la combinaison oxyde de polyéthylène/astringent.

5. Un produit d'aide au rasage selon la revendication 4, caractérisé en ce que la structure de retenue est un matériau thermoplastique insoluble dans l'eau.

6. Un produit d'aide au rasage selon l'une quelconque des revendications précédentes, qui est en forme de bande.

7. Une enveloppe de lame de rasoir caractérisé en ce que ladite enveloppe est munie d'un produit d'aide au rasage selon l'une quelconque des revendications précédentes.

8. Une enveloppe de lame de rasoir selon la revendication 7, caractérisé en ce que le produit d'aide au rasage est prévu sur une surface supérieure de l'enveloppe.

9. Une tête de rasage caractérisée par une enveloppe de lame, au moins une lame, la ou chaque lame comportant un bord de coupe de lame, et un support de lame muni d'une barre de protection disposée vers l'avant du bord de coupe de lame, dans lequel l'enveloppe de lame et/ou la barre de protection est munie d'un produit d'aide au rasage selon l'une quelconque des revendications 1 à 6.

10. Un procédé de réalisation d'un produit d'aide au rasage selon l'une quelconque des revendications 1 à 6, comprenant les étapes consistant à former un mélange d'un matériau thermoplastique, d'oxyde de polyéthylène et d'un astringent, et à soumettre ledit mélange à une étape de formage choisie parmi l'extrusion, le moulage par injection et le moulage par compression, afin de fabriquer un véhicule de distribution qui comporte une structure microporeuse en matériau thermoplastique contenant l'oxyde de polyéthylène et l'astringent.

**Patentansprüche**

1. Festes und zum Anbringen an einen Rasiererkopf (10) passendes Rasierhilfsmittel (24) mit einem Polyethylenoxid-Rasiergleitmittel, dadurch gekennzeichnet, daß als wasserlösliches Adstringens ein dreiwertiges Aluminiumsalz zugegeben wird, so daß durch das Rasierhilfsmittel sichtbare Zeichen für (Rasier-)Schnitte oder Kratzer wesentlich vermindert werden und gleichzeitig das mit der Bereitstellung eines Adstringens alleine einhergehende Unbehagen beseitigt wird.

2. Rasierhilfsmittel nach Anspruch 1, dadurch gekennzeichnet, daß das Adstringens aus Aluminiumsulfat besteht.

3. Rasierhilfsmittel nach Anspruch 1 oder 2, dadurch gekennzeichnet, daß das Adstringens in einer Menge von nicht mehr als etwa 20 Gew.-% des Rasierhilfsmittels vorhanden ist.

4. Rasierhilfsmittel nach einem der vorhergehenden Ansprüche, zusätzlich gekennzeichnet durch eine mikroporöse Rückhaltestruktur, die als Abgabevehikel für die Polyethylenoxid/Adstringens-Kombination dient.

5. Rasierhilfsmittel nach Anspruch 4, dadurch gekennzeichnet, daß die Rückhaltestruktur aus einem wasserunlöslichen thermoplastischen Material besteht.

6. Rasierhilfsmittel nach einem der vorhergehenden Ansprüche in Form eines Streifens.

7. Rasierklingenabdeckung, dadurch gekennzeichnet, daß die Abdeckung mit einem Rasierhilfsmittel nach einem der vorhergehenden Ansprüche versehen ist.

**8.** Rasierklingenabdeckung nach Anspruch 7, dadurch gekennzeichnet, daß sich das Rasierhilfsmittel auf einer Oberseite der Abdeckung befindet.

**9.** Rasierkopf, gekennzeichnet durch eine Klingenabdeckung, mindestens eine Klinge mit jeweils einer (Klingen)schneidkante und einem Klingenhalter mit einer vor der (Klingen)schneidkante befindlichen Schutzleiste, wobei die Klingenabdeckung und/oder die Schutzleiste mit einem Rasierhilfsmittel nach einem der Ansprüche 1 bis 6 versehen ist.

**10.** Verfahren zur Herstellung eines Rasierhilfsmittels nach einem der Ansprüche 1 bis 6 durch Ausbilden eines Gemischs aus einem thermoplastischen Material, von Polyethylenoxid und einem Adstringens und Ausformen des Gemischs durch Strangpressen, Spritzguß oder Formpressen zur Herstellung eines Abgabevehikels, umfassend eine mikroporöse Struktur aus thermoplastischem Material mit dem Polyethylenoxid und dem Adstringens.

# FIG-1